# EUROPEAN PATENT SPECIFICATION

(11) **EP 2 878 305 B1**
(45) Date of publication and mention of the grant of the patent: **19.07.2017**
(21) Application number: 13793632.4
(22) Date of filing: 21.05.2013
(51) Int. Cl.: A61K 36/537, A61P 37/00

(54) **PHARMACEUTICAL COMPOSITION FOR USE IN PREVENTING OR TREATING STAT3-MEDIATED DISEASE, COMPRISING SALVIA PLEBEIA R. BR. EXTRACT OR FRACTION THEREOF AS ACTIVE INGREDIENT COMPOSITION**
PHARMAZEUTISCHE ZUSAMMENSETZUNG ZUR VERWENDUNG ZUR VORBEUGUNG ODER BEHANDLUNG VON STAT3-VERMITTELTEN ERKRANKUNGEN MIT EINEM SALVIA PLEBEIA-R. BR.- EXTRAKT ODER EINEM TEIL DAVON ALS WIRKSTOFF
COMPOSITION PHARMACEUTIQUE POUR L'UTILISATION DANS LA PRÉVENTION OU LE TRAITEMENT DE MALADIES MÉDIÉES PAR STAT-3, CONTENANT UN EXTRAIT OU UNE FRACTION DE SALVIA PLEBEIA R. BR. EN TANT QUE PRINCIPE ACTIF

(30) Priority: 21.05.2012 KR 20120053899
(43) Date of publication of application: 03.06.2015
(73) Proprietor: Korea Research Institute of Bioscience and Biotechnology, Daejeon 305-806 (KR)
(72) Inventor: RHO, Mun Chual, Daejeon 305-806 (KR); OH, Hyun Mee, Daejeon 305-806 (KR); LEE, Woo Song, Daejeon 305-806 (KR); KIM, Sang Hyun, Daegu 700-842 (KR); LEE, Seung Woong, Daejeon 305-806 (KR); PARK, Chan Sun, Daejeon 305-806 (KR); AHN, Keug Hyun, Daejeon 305-806 (KR)
(74) Representative: Andrews, Robert
(86) International application number: PCT/KR2013/004457
(87) International publication number: WO 2013/176471

(56) References cited:
- KR-A- 20110 078 672
- KR-B1- 100 813 483
- QU X J ET AL: "Protective effects of Salvia plebeia compound homoplantaginin on hepatocyte injury", FOOD AND CHEMICAL TOXICOLOGY, PERGAMON, GB, vol. 47, no. 7, 1 July 2009 (2009-07-01), pages 1710-1715, XP026157673, ISSN: 0278-6915, DOI: 10.1016/J.FCT.2009.04.032 [retrieved on 2009-05-03]
- JIN X F ET AL: "Chemical fingerprint and quantitative analysis of Salvia plebeia R.Br. by high-performance liquid chromatography", JOURNAL OF PHARMACEUTICAL AND BIOMEDICAL ANALYSIS, NEW YORK, NY, US, vol. 48, no. 1, 10 September 2008 (2008-09-10), pages 100-104, XP024098168, ISSN: 0731-7085, DOI: 10.1016/J.JPBA.2008.05.027 [retrieved on 2008-05-28]
- JO, SUN YOUNG ET AL.: 'A Study on the Anti-inflammatory and Anti-allergic Effect of Salvia plebeia R. extracts' KOREAN SOCIETY OF PHARMACOGNOSY vol. 41, no. 1, 2010, pages 31 - 37, XP053014167
- SHIN, T.-Y. ET AL.: 'Effect of Salvia plebeia on IgE antibody mediated allergic reaction in rats' INTERNATIONAL JOURNAL OF ORIENTAL MEDICINE vol. 1, no. 2, 2000, pages 29 - 35, XP053006177
- JUNG, H.-J. ET AL.: 'Anti-inflammatory, anti-angiogenic and anti-nociceptive activities of an ethanol extract of Salvia plebeia R. Brown' JOURNAL OF ETHNOPHARMACOLOGY vol. 126, 2009, pages 355 - 360, XP026718082

## Description

### Technical Field

The present invention relates, in general, to a pharmaceutical composition for use in preventing or treating signal transducers and activators of transcription 3 (hereinafter, "STAT3")-mediated diseases, containing a *Salvia plebeia* R. Br. extract or fraction thereof as an active wherein the STAT3-mediated disease is osteoporosis, rheumatoid arthritis, atopic dermatitis or Crohn's disease. Furthermore, the present invention relates to a food composition and a quasi-drug composition for use in preventing or improving STAT3-mediated diseases, containing the *Salvia plebeia* R. Br. extract or fraction thereof as an active ingredient, wherein the STAT3-mediated disease is osteoporosis, rheumatoid arthritis, atopic dermatitis or Crohn's disease.

### Background Art

Generally, an interleukin-6 (IL-6) is a cytokine also referred to as B cell stimulatory factor (BSF2) or interferon-2 (INF-2), and was discovered as a differentiation factor involved in activation of B lymphocytes. Since then, IL-6 has been known as a multi-functional cytokine capable of affecting functions of various cells. IL-6 delivers its biological activities mediated by two different kinds of proteins on a cell membrane. One of the proteins serves as an IL-6 receptor, which is a membrane-bound protein expressed through a cell membrane and has a molecular weight of about 80 kDa, whereas the other protein is a membrane protein gp130 with a molecular weight of about 130 kDa, which is involved in signal transduction of a non-ligand binding IL-6 and IL-6 receptor together form an IL-6/IL-6 receptor complex, which then binds to gp130. Upon binding between ligands and receptors, Janus Kinase 2 (JAK2) is activated by transphosphorylation. The activated JAK2 phosphorylates various tyrosine residues in cytoplasmic domains of the receptors causing them to serve as docking sites for the intracelluar proteins such as SH2 or STAT3 having phosphotyrosine binding motifs. The STAT3 bound to the cytoplasmic domain of a given receptor is released from the receptor after being phosphorylated by JAK2. The thus activated STAT3s bind to each other to form homodimers or heterdimers, enter a nucleus, and bind to the recognition sequence of a target gene thereby increasing transcription.

The signal transduction system induced by IL-6 has been already known to be associated with inflammatory diseases, autoimmune diseases, and metabolic diseases. Research on the inhibition of the signal transduction system induced by IL-6 has been actively carried out for the treatment of inflammatory diseases, autoimmune diseases, etc. Of them, the inhibition of IL-6 induced signal transduction system using anti-IL-6 receptor antibody (IL-6 R antibody) has been most well known. For example, a synovial cell growth inhibitor for rheumatoid arthritis remedy using IL-6 R antibody (WO Publication 96/011020), and treatment of plasmacytosis, hyperimmunoglobulinemia, anemia, nephritis, cachexia, rheumatoid arthritis, livestock-farmer disease and mesangium proliferative nephritisinduced by IL-6 products using anti IL-6 R antibody has been already known (WO Publication 96/012503). Additionally, anti IL-6 R antibody has been reported to be applicable to the treatment of multiple sclerosis, uveitis, chronic thyroiditis, and sensitized T-cell related diseases such as delayed hypersensitivity and atopic dermatitis (WO Publication 98/042377), treatment of systemic erythematosus, and treatment of Crohn's disease (WO Publication 99/047170), treatment of pancreatitis (WO Publication 00/010607), treatment of psoriasis (WO Publication 02/034292), and treatment of juvenile chronic arthritis (WO Publication 02/080969). However, when the anti IL-6 R antibody is introduced into a subject it may have an epitope which may be perceived as a foreign protein by the subject and thus it may be still immunogenicity. Accordingly, in order to solve the above problem, numerous studies have been conducted to develop therapeutic agents mediated by IL-6 and STAT3 using small molecule compounds not recognized by immune systems instead of proteins.

Meanwhile, interleukin-11 (IL-11) is a proinflammatory cytokine belonging to IL-6 family and has a signal transduction system similar to that of IL-6, and has been known to increase its expression in hematopoietic cells, immune response cells, inflammatory cells and various cancer cells. Recently, IL-11 was reported to bind to its receptors, IL-11R and gp130, thereby promoting proliferation of stomach cancer and colorectal cancer cell, and cancer cell invasion *(*Nakayama T et al., Int J Oncol, 2007, 30, 825-833; Yoshizaki A et al., Int J Oncol, 2006, 29, 869-876), and when smad7 is activated by IL-11/STAT3 signal and TGF signal-inducing smad activator are blocked at the same time, an oncogenic program associated with an anti-apoptotic gene, a proangiogenic gene, or proliferation gene, may be activated thereby inducing inflammation-related gastric tumor *(*Ernst et al., J Clin. Invest, 2008, 118 (5), 1728-1738). Additionally, IL-11, being involved in proliferation and regulation of differentiation of osteocytes, has been known to be a treatment target in osteoporosis *(*Sims NA el al., J Bone Miner Res. 2005, Jul; 20(7):1093-102.). Based on the above reports, a gp130/JAK/STAT3 pathway by IL-11 has emerged as a new target in the treatment of various diseases, including osteoporosis.

### Disclosure

### Technical Problem

The inventors of the present invention, while endeavoring to find a natural substance targeting the STAT3 pathway activated by IL-6 or IL-11, discovered that an extract or fraction of *Salvia plebeia* R. Br. inhibits the transcription activity and phosphorylation of STAT3, which is a transcription factor associated with inflammation and autoimmune diseases activated by IL-6. It also effectively inhibits the signal transduction pathway induced by IL-6, thereby completing the present invention relating to a pharmaceutical composition for use in preventing or treating STAT3-mediated diseases containing the extract or fraction of *Salvia plebeia* R. Br. as an active ingredient.

### Technical Solution

An objective of the present invention is to provide a pharmaceutical composition for use in preventing or treating STAT3-mediated diseases containing the extract or fraction of *Salvia plebeia* R. Br. as an active ingredient, wherein the STAT3-mediated disease is osteoporosis, rheumatoid arthritis, atopic dermatitis or Crohn's disease.

Disclosed is a method for treating STAT3-mediated diseases including administering the above pharmaceutical composition to a subject suspected of having the STAT3-mediated disease(s).

A further objective of the present invention is to provide a food composition for use in preventing or improving STAT3-mediated diseases containing the extract or fraction of *Salvia plebeia* R. Br. as an active ingredient, wherein the STAT3-mediated disease is osteoporosis, rheumatoid arthritis, atopic dermatitis or Crohn's disease.

A still further objective of the present invention is to provide a quasi-drug composition for use in preventing or improving STAT3-mediated diseases containing the extract or fraction of *Salvia plebeia* R. Br. as an active ingredient, wherein the STAT3-mediated disease is osteoporosis, rheumatoid arthritis, atopic dermatitis or Crohn's disease.

### Advantageous Effects

The extract or fraction of *Salvia plebeia* R. Br. for use according to the present invention is derived from a natural substance which has long been used as a natural medicine and thus has no side effects, and enabling it to effectively inhibit the signal transduction system of STAT3 induced by IL-6. Thus it is for use in the prevention or treatment of STAT3-mediated diseases, wherein the STAT3-mediated disease is osteoporosis, rheumatoid arthritis, atopic dermatitis or Crohn's disease.

### Description of Drawings

FIG. 1 is a graph illustrating the inhibitory effects of an ethanol extract of *Salvia plebeia* R. Br. and an ethyl acetate fraction of *Salvia plebeia* R. Br. on the expression of luciferase induced by IL-6.
FIG. 2 is a picture illustrating the inhibitory effect of an ethanol extract of *Salvia plebeia* R. Br. on STAT phosphorylation induced by IL-6.
FIG. 3 is a picture illustrating the inhibitory effect of an ethyl acetate fraction of *Salvia plebeia* R. Br. on STAT phosphorylation induced by IL-6.
FIG. 4 is a picture illustrating the inhibitory effects of an ethanol extract of *Salvia plebeia* R. Br. and an ethyl acetate fraction of *Salvia plebeia* R. Br. on JAK2 and ERK phosphorylations induced by IL-6.
FIG. 5 is a graph illustrating the inhibitory effect of an ethanol extract of *Salvia plebeia* R. Br. on the expression of SOCS-3 mRNA induced by STAT3.
FIG. 6 is a graph illustrating the inhibitory effect of an ethanol extract of *Salvia plebeia* R. Br. on the expression of MCP-1 mRNA induced by STAT3.
FIG. 7 is a graph illustrating the inhibitory effect of an ethanol extract of *Salvia plebeia* R. Br. on the expression of ICAM-1 mRNA induced by STAT3.
FIG. 8 is a diagram illustrating a schedule for constructing a mouse model with rheumatoid arthritis for confirming the efficacy of an ethanol extract of *Salvia plebeia* R. Br.
FIG. 9 is a graph illustrating administering an ethanol extract of *Salvia plebeia* R. Br. reduces the level of the paw thickness of a rheumatoid arthritis-induced mouse.
FIG. 10 is a graph illustrating administering an ethanol extract of *Salvia plebeia* R. Br. alleviates severity of arthritis of a rheumatoid arthritis-induced mouse.
FIG. 11 is a graph illustrating administering an ethanol extract of *Salvia plebeia* R. Br. reduces the recurrence rate of arthritis of a rheumatoid arthritis-induced mouse.
FIG. 12 is a graph illustrating administering an ethanol extract of *Salvia plebeia* R. Br. reduces the level of immunoglobulin G 1 in blood of a rheumatoid arthritis-induced mouse.
FIG. 13 is a graph illustrating administering an ethanol extract of *Salvia plebeia* R. Br. reduces in the level of immunoglobulin G2a in blood of a rheumatoid arthritis-induced mouse.
FIG. 14 is a diagram illustrating a schedule for constructing a mouse model with atopic dermatitis.
FIG. 15 is a graph confirming that an ethanol extract of *Salvia plebeia* R. Br. reduces ear thickness of an atopic dermatitis-induced mouse, wherein AD represents atopic dermatitis, and MEBC represents a *Salvia plebeia* R. Br. extract.
FIG. 16 is a graph confirming that an ethanol extract of *Salvia plebeia* R. Br. inhibits the blood level of immunoglobulin E in an atopic dermatitis-induced mouse.
FIG. 17 is a graph confirming that an ethanol extract of *Salvia plebeia* R. Br. inhibits the blood level of immunoglobulin G2a in an atopic dermatitis-induced mouse.
FIG. 18 is a graph illustrating that an ethanol extract of *Salvia plebeia* R. Br. inhibits the blood level of histamine in blood of an atopic dermatitis-induced mouse.
FIG. 19 is a graph illustrating that an ethanol extract of *Salvia plebeia* R. Br. inhibits the secretion of proinflammatory cytokines of an atopic dermatitis-induced mouse, wherein AD represents atopic dermatitis, and MEBC represents a *Salvia plebeia* R. Br. extract.

### Best Mode

In an aspect of the present invention, there is provided a pharmaceutical composition for use in preventing or treating STAT3-mediated diseases containing a *Salvia plebeia* R. Br. extract or fraction thereof as an active ingredient, wherein the STAT3-mediated disease is osteoporosis, rheumatoid arthritis, atopic dermatitis or Crohn's disease.

As used herein, the term *"Salvia plebeia* R. Br." refers to a biennial dicotyledonous plant belonging to *Lamiaceae* Family, *Lamiales* Order, also called *Ocimum virgatum .* Morphologically, it has a rectangular columnar stem covered with short and smooth hairs, ovate or lanceolate leaves with blunt ends or sudden sharp edges, and a circular or wedge-shaped base. From the pharmacological aspect, *Salvia plebeia* R. Br. has been known effective in the treatment of the bleeding of cough, hematemesis, hematuria, ascites, nebula, etc., but it has not been known anything relating to the treatment of STAT3-mediated diseases. Additionally, *Salvia plebeia* R. Br. may be purchased from a commercial market or those collected from or cultivated in the field may be used.

As used herein, the term *"Salvia plebeia* R. Br. extract" refers to an extract obtained by extracting *Salvia plebeia* R. Br. The *Salvia plebeia* R. Br. extract may be eluted out by adding about 2 - 20 times, preferably about 3 - 5 volumes of water; a polar solvent such as C₁ - C₆ alcohol such as methanol, ethanol, and butanol; or a mixed solvent thereof mixed in a ratio of about 1:0.1 - 1:10, relative to that of the dry weight of the pulverized *Salvia plebeia* R. Br., at an extraction temperature of 20°C - 100°C, preferably at room temperature, for an extraction period of about 12 hours - 4 days, preferably for 3 days, via hot-water extraction, cold extraction, reflux extraction, sonication extraction, etc. Preferably, the extraction may be performed 1- 5 times in a continuous fashion via cold extraction, and the resultant is filtered under reduced pressure, and the resulting filtrate is concentrated under reduced pressure at 20°C - 100°C via rotary vacuum evaporator, preferably at room temperature, and thereby obtain a crude *Salvia plebeia* R. Br. extract soluble in water, alcohols, or a mixed solvent thereof, but is not limited thereto. It may include any liquid extract, a diluent or concentrate of the liquid extract, a dry product obtained by drying the liquid extract, or a crude purified product or a purified product as long as they are capable of exhibiting therapeutic effects on treatment of STAT3-mediated diseases of the present invention. The *Salvia plebeia* R. Br. extract may be obtained from various organs of natural *Salvia plebeia* R. Br, or hybrids or variants of *Salvia plebeia* R. Br., for example, roots, above-arial parts, stems, leaves, flowers, main bodies of fruits, skins of fruits, and also from cultured products of the plant tissues of *Salvia plebeia* R. Br.

In an embodiment of the present invention, the *Salvia plebeia* R. Br. extract may be obtained from the pulverized *Salvia plebeia* R. Br. using ethanol as an elution solvent, via cold extraction at room temperature for 7 days, followed by filtration under reduced pressure, and removal of the ethanol solvent via rotation vacuum evaporator (Example 1).

As used herein, the term "fraction" refers to a product obtained as a result of fractionation of a particular component or particular group from a mixture composed of various components. Preferably, the fraction may be a fractionated product obtained via solvent fractionation of the *Salvia plebeia* R. Br. extract using a solvent such as n-hexane, ethyl acetate, etc., including both polar fractions and nonpolar fractions, specifically, hexane fraction, an ethyl acetate fraction, etc. More preferably, the fraction may be an ethyl acetate fraction, and may include any fraction capable of exhibiting therapeutic effects for the treatment of STAT3-mediated diseases of the present invention, but is not limited thereto. Preferably, the water fraction, for not inhibiting the activity of STAT3 luciferase, may not be included in the fractions of the present invention.

A nonpolar solvent soluble layer may be obtained by suspending the crude extract of *Salvia plebeia* R. Br. obtained above in distilled water, adding about 1-100 times, preferably about 1- 5 volumes of a nonpolar solvent such as ethyl acetate, relative to that of the suspension, followed by 1-10 times, preferably 2 - 5 times, of extractions and separations.

Additionally, a further conventional fractionation may be performed (Harbome J.B. Phytochemical methods: A guide to modern techniques of plant analysis, 3rd Ed. p6-7, 1998). Specifically, each of the solvent soluble extracts of *Salvia plebeia* R. Br. may be obtained by suspending the above crude extract of *Salvia plebeia* R. Br. in water, and then performing consecutive extractions by subjecting the resultant to an equal amount of ethyl acetate used as a solvent. More specifically, an ethyl acetate soluble fraction and a water soluble fraction may be obtained by suspending the crude extract of *Salvia plebeia* R. Br. in water followed by adding an equal amount of ethyl acetate thereto.

In an embodiment of the present invention, an ethyl acetate soluble fraction was obtained by suspending the crude extract of *Salvia plebeia* R. Br. in water, adding an equal amount of ethyl acetate thereto to thereby mix and fractionate, repeating the above process 3 times, and concentrating the resulting ethyl acetate soluble fraction under reduced pressure (Example 1).

As used herein, the term "STAT3-mediated diseases" collectively refers to diseases occurring when interleukin (IL)-6 or IL-11 binds to their receptors thereby inducing phosphorylation of STAT3, i.e., activation of STAT3, where the phosphorylated STAT3 moves into a nucleus thereby inducing expression of the target genes involved in the occurrence or progress of autoimmune diseases, inflammatory diseases, or metabolic diseases. The STAT3-mediated diseases, although not particularly limited thereto, may be autoimmune diseases, inflammatory diseases, or metabolic diseases.

As used herein, the term "autoimmune diseases" collectively refers to diseases where immune responses of a morbid subject to self-antigens become direct or indirect causes, and in an objective of the present invention, may refer to autoimmune diseases mediated by STAT3 activated by IL-6 or IL-11. The autoimmune diseases, although not limited thereto, may be atopic dermatitis, rheumatoid arthritis, osteoarthritis, psoriasis, asthma, graft-versus-host disease (GVHD), immune deficiency syndrome, systemic lupus erythematosus, or multiple sclerosis. Since the activated STAT3 acts as a major transcription factor in autoimmune diseases the composition of the present invention capable of inhibiting the activation of STAT3 mediated by IL-6 will be useful for the treatment of autoimmune diseases.

As used herein, the term "inflammatory diseases" collectively refers to diseases which accompany inflammation as a major symptom, and may refer to inflammatory diseases mediated by STAT3 activated by IL-6 or IL-11. The inflammatory diseases include plasmacytosis, hyperimmunoglobulinemia, anemia, nephritis, cachexia, livestock-farmer disease, mesangium proliferative nephritis, uveitis, chronic thyroiditis, delayed hypersensitivity, Crohn's disease, pancreatitis, juvenile idiopathic arthritis, diabetes, and Alzheimer's disease. A pharmaceutical composition containing a *Salvia plebeia* R. Br. extract or fraction thereof may be useful for the treatment of inflammation-induced diseases but is more useful for the treatment of body regions other than the skin areas with induced inflammations mediated by STAT3. IL-6 has been known as a cytokine involved in various immune responses but its role in skins appears to be different. In detail, in host defense in skin, IL-6 is involved in the protection of skin from stimulation or inflammation by participating in the enhancement of *stratum corneum,* and requires other factors such as interferon gamma in other roles, thus differing in its roles when it is involved in immune responses in other areas such as intestinal organs. Accordingly, as for inflammatory diseases, IL-6 may be more useful in the treatment of inflammations in body regions other than those on skin.

As used herein, the term "metabolic diseases" collectively refers to diseases caused by metabolic disorders in a living organism, and may refer to metabolic diseases mediated by STAT3 activated by IL-6 or IL-11. The metabolic diseases, although not limited thereto, may be osteoporosis, arteriosclerosis, or myocardial infarction. One of the causes for osteoporosis may be excess production of IL-6 that promotes bone absorption. Accordingly, the composition for use according to the present invention, capable of inhibiting the activity of STAT3 mediated by IL-6, is effective in treating osteoporosis.

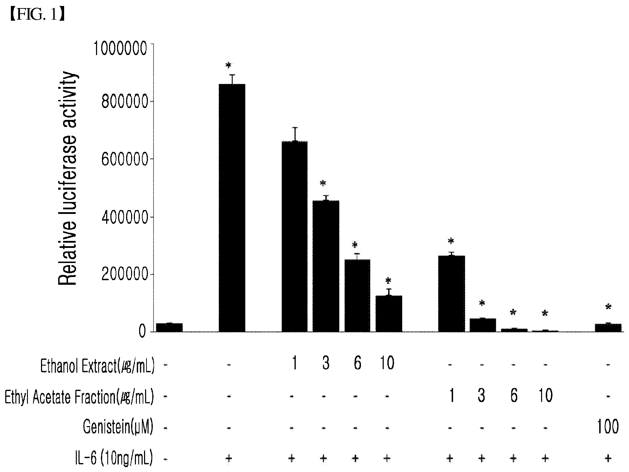

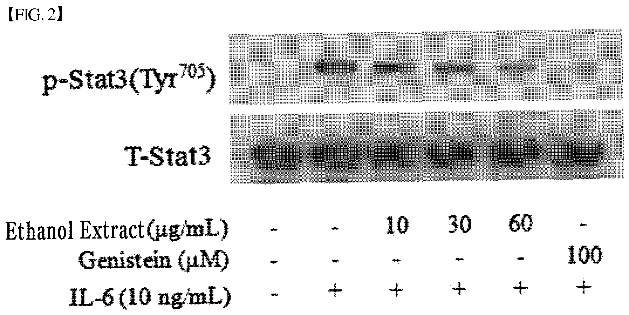

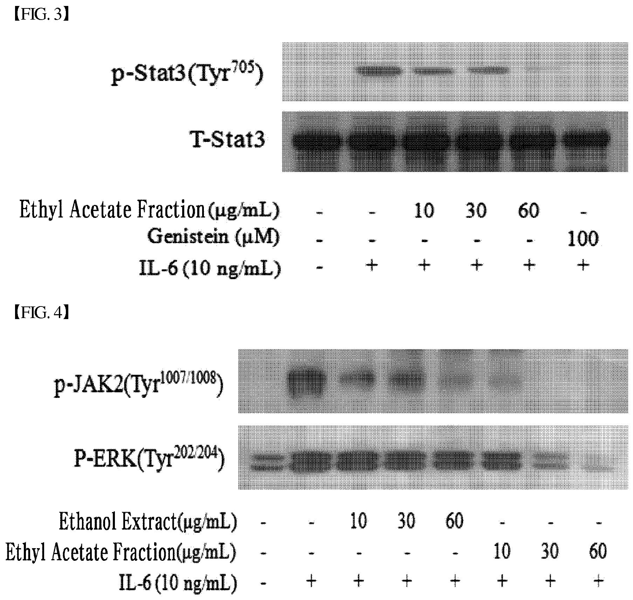

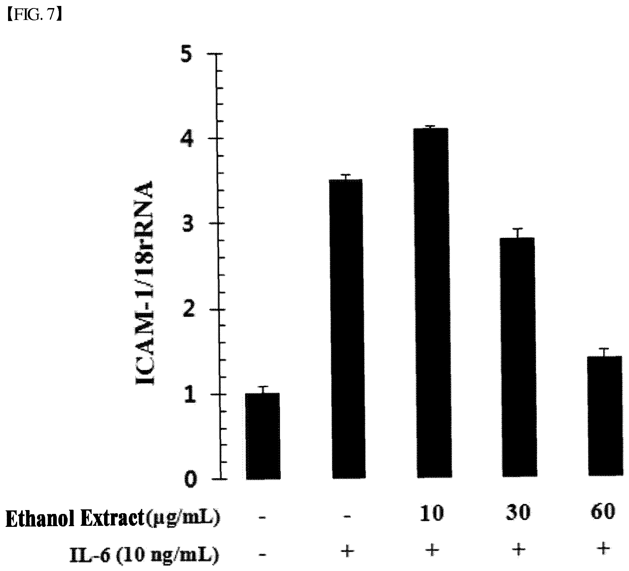

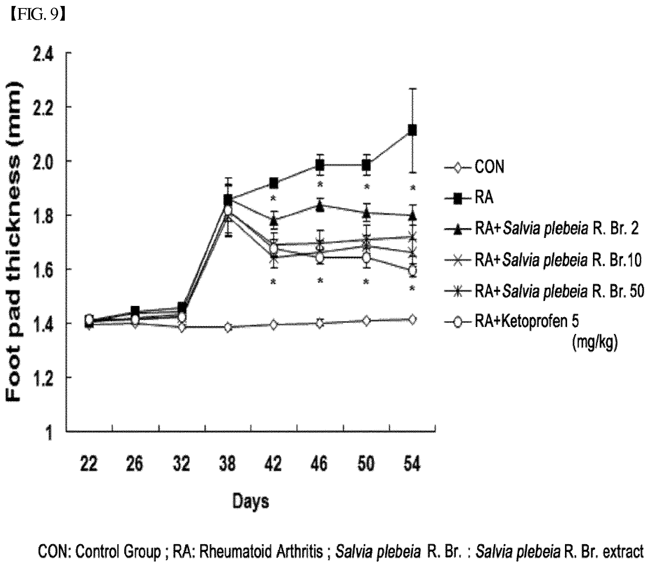

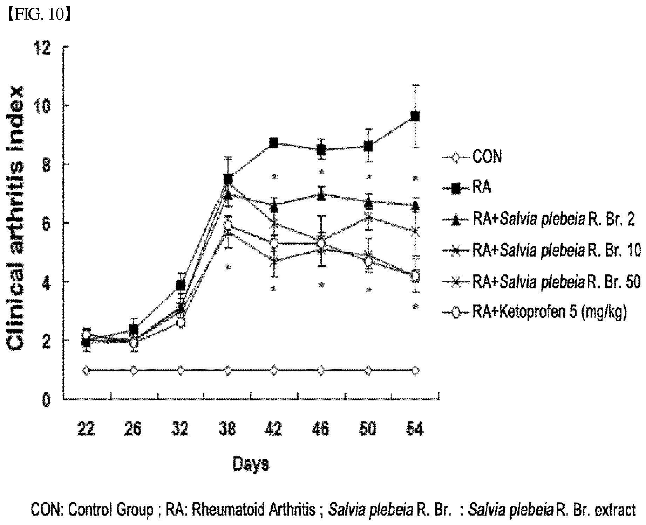

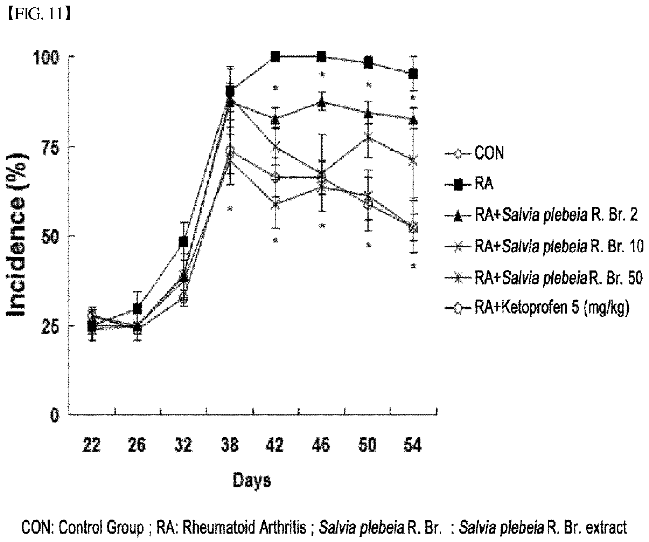

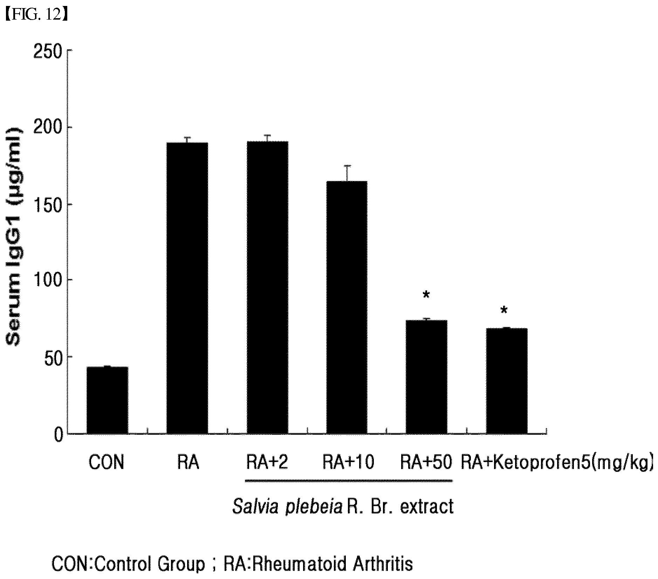

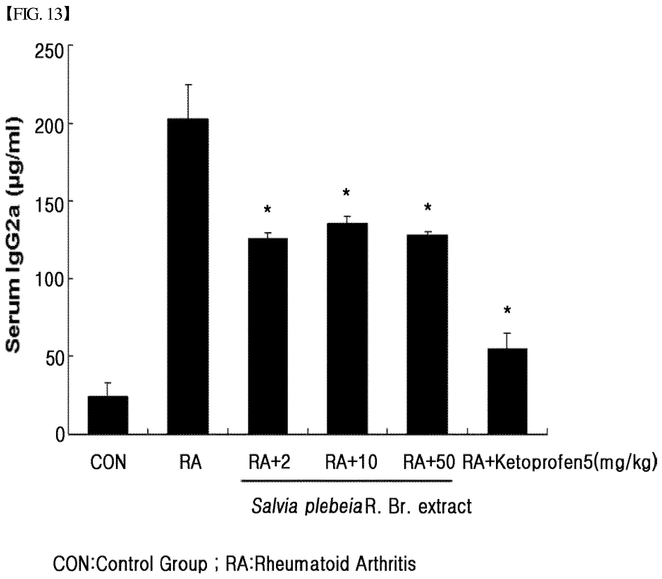

In an embodiment of the present invention, the present inventors confirmed that an ethanol extract of *Salvia plebeia* R. Br. and an ethyl acetate fraction thereof can inhibit the activity of STAT3 luciferase induced by IL-6 in a concentration-dependent manner (FIG. 1). Additionally, they also confirmed that the ethanol extract of *Salvia plebeia* R. Br. and the ethyl acetate fraction thereof effectively can inhibit the phosphorylation of the 705^{th} tyrosine residue (Tyr⁷⁰⁵) of STAT3 induced by IL-6 in a concentration-dependent manner (FIGS. 2 and 3). Furthermore, they also confirmed that the ethanol extract of *Salvia plebeia* R. Br. and the ethyl acetate fraction thereof can also effectively inhibit the phosphorylation of JAK2, which mediates the phosphorylation of STAT3, and that the ethanol extract of *Salvia plebeia* R. Br. can reduce the mRNA expressions of SOCS-3, MCP-1, and ICAM-1, which are the target gens of STAT3 (FIG. 7). Additionally, the present inventors confirmed that the ethanol extract of *Salvia plebeia* R. Br. can effectively treat paw edema, occurrence of arthritis, etc., of mice with rheumatoid arthritis induced by type II collagen, and also that, by comparison of the efficacies of the ethanol extract of *Salvia plebeia* R. Br. with Ketoprofen, a commercial therapeutic agent for treating arthritis, it showed a similar therapeutic efficacy to that of Ketoprofen (FIGS. 9,10 & 11). Furthermore, the ethanol extract of *Salvia plebeia* R. Br. exhibited an effective inhibitory activity against the production of immunoglobulins G1 and G2a specific to collagen (FIGS. 12 & 13). From the foregoing, it was confirmed that the *Salvia plebeia* R. Br. extract can effectively prevent or treat rheumatoid arthritis, a representative autoimmune disease, and thus confirmed that it can be useful for the treatment of STAT-3 mediated diseases such as autoimmune diseases.

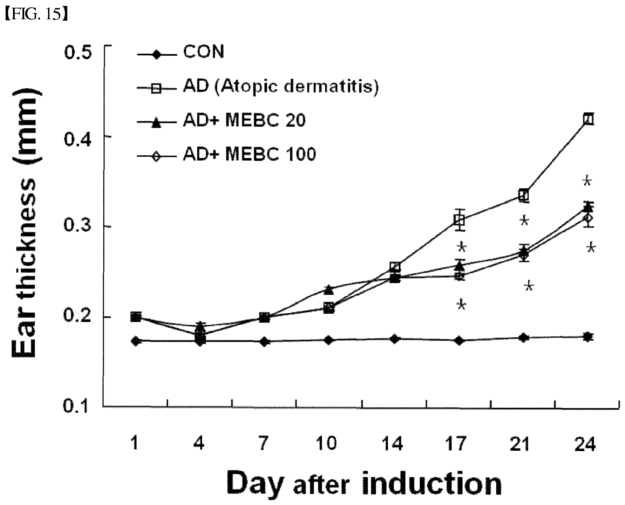

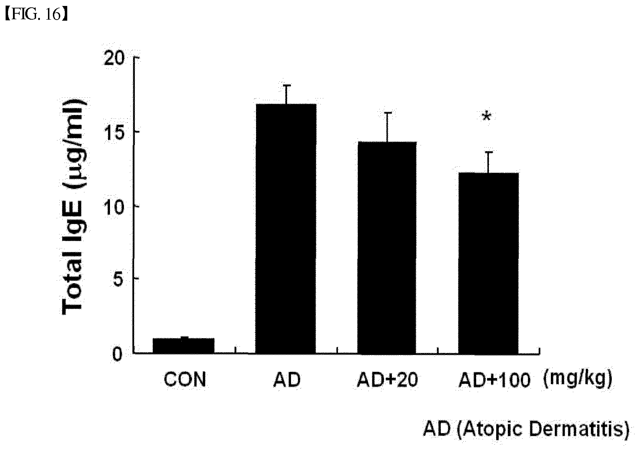

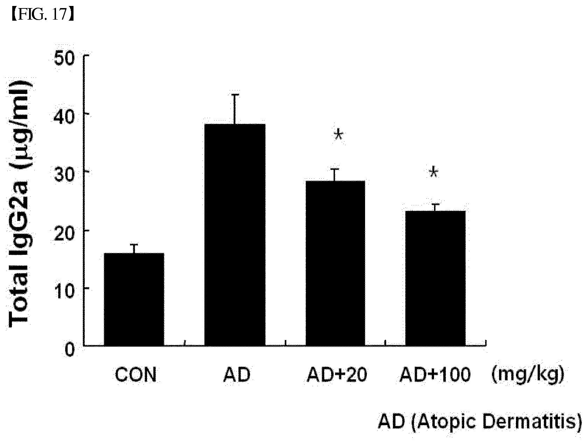

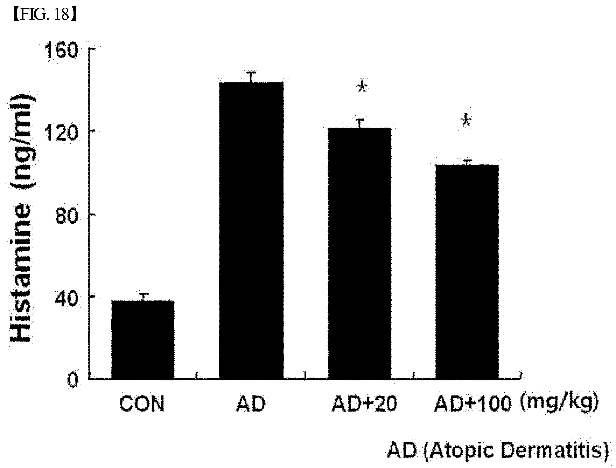

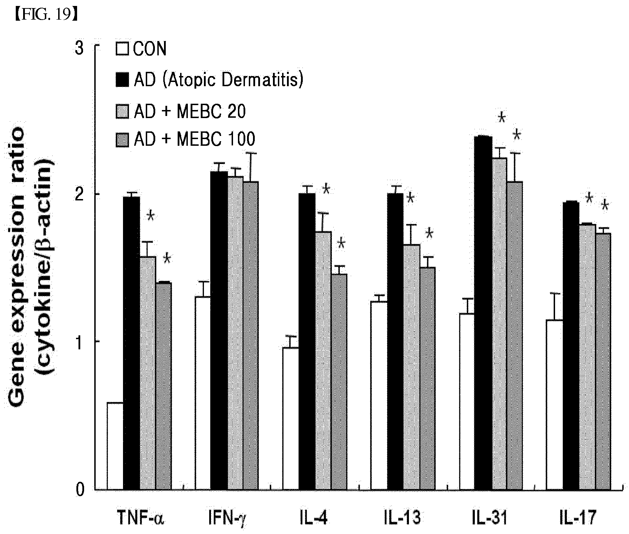

Additionally, the present inventors confirmed that the *Salvia plebeia* R. Br. extract has a therapeutic effect in a mouse model with atopic dermatitis induced by a dust mite extract and DCNB. In detail, the ethanol extract of *Salvia plebeia* R. Br. effectively reduced the ear thickness edema in an atopic dermatitis-induced mouse (FIG. 15), and also exhibited the inhibitory effect against the production of immunoglobulins E and G2a in blood due to atopic dermatitis (FIGS. 16 & 17). Additionally, the ethanol extract of *Salvia plebeia* R. Br. showed an inhibitory effect against histamine release due to atopic dermatitis (FIG. 18), and also an inhibitory effect against production and secretion of inflammatory cytokines (FIG. 19). From the above results, it was confirmed that the *Salvia plebeia* R. Br. extract can effectively prevent or treat atopic dermatitis, a kind of representative autoimmune diseases, and thus, can be useful for the treatment of STAT-3 mediated diseases such as autoimmune diseases. In conclusion, the *Salvia plebeia* R. Br. extract or fraction thereof capable of inhibiting the activity of STAT3 induced by IL-6, can be effectively used for the prevention or treatment of STAT3-mediated diseases.

As used herein, the term "prevention" refers to all kinds of activities associated with inhibition or delay of STAT3-mediated diseases by the administration of the above composition, and "treatment" refers to all kinds of activities associated with improvement or advantageous changes in the symptoms of STAT3-mediated diseases by the administration of the above composition.

The pharmaceutical composition for use according to the present invention containing a *Salvia plebeia* R. Br. extract or fraction thereof may further contain a suitable carrier, excipient, or diluent conventionally used in the manufacture of pharmaceutical compositions. In particular, the amount of the *Salvia plebeia* R. Br. extract or fraction thereof according to the present invention, although not particularly limited, is preferably contained in the amount of 0.0001 - 10 wt%, more preferably 0.001- 1 wt%, relative to the total weight of the composition.

The above pharmaceutical composition may be prepared in any one formulation selected from the group consisting of tablets, pills, powders, granules, capsules, suspensions, liquid medicine for internal use, emulsions, syrups, sterile aqueous solutions, non-aqueous solvents, suspensions, emulsions, lyophilized preparations, and suppositories, and may be a formulation for oral administration or others for various parenteral administration routes. For formulations, the conventional fillers, extenders, binders, humectants, disintegrating agents, diluents such as surfactants, or excipients, may be used. For solid formulations for oral administration, tablets, pills, powders, granules, capsules, etc., may be included. The solid formulations may be prepared by mixing at least one excipient, e.g., starch, calcium carbonate, sucrose, or lactose, gelatin, etc., with at least one compound. Additionally, lubricants such as magnesium stearate, talc, etc., may be used in addition to simple excipients. Examples of liquid formulations for oral administration may include suspensions, liquid medicine for internal use, emulsions, syrups, etc., and may include various excipients, e.g., humectants, sweeteners, fragrants, preservatives, etc., in addition to simple diluents such as water and liquid paraffin. Examples of parenteral formulations may include sterile aqueous solutions, non-aqueous solvents, suspensions, emulsions, lyophilized preparations, and suppositories. Examples of non-aqueous solvents, suspending agents, may include propylene glycol, polyethylene glycol, vegetable oils such as olive oil, an injectable ester such as ethyl oleate, etc. Examples of substrates to be used for suppositories may include witepsol, microgol, tween 61, cacao butter, laurinum, glycerogelatin, etc.

The composition for use according to the present invention may be administered in a pharmaceutically effective amount.

As used herein, the term "pharmaceutically effective amount" refers to an amount sufficient for treating a disease with a reasonable benefit/risk ratio applicable to medical treatment, and the level of effective dose may be determined based on factors including type of a subject, age and sex of a subject, type and severity of disease(s), activity of drug, sensitivity to drug, duration of administration, routes of administration and drug efflux rate, duration of treatment, drugs used in combination, and other factors well known in the medical field. The composition for use according to the present invention may be administered as an individual therapeutic agent or in combination with other therapeutic agents, may be administered in sequential order or simultaneously with other therapeutic agents, and may be administered as a single dose or a multi-dose. It is important that the administration be performed in light of all the above-described factors so that a maximum effect can be obtained with the least amount without any adverse effect, and can be easily determined by a skilled person in the art. The preferable amount of the composition of the present invention to be administered may vary depending on the physical state and body weight of a subject, severity of disease(s), type of drug, and routes and duration of administration. However, preferably, the *Salvia plebeia* R. Br. extract or fraction thereof according to the present invention may be administered in the amount of 0.0001 - 100 mg/kg daily, and more preferably, 0.001 - 100 mg/kg. The administration may be performed once daily or a few times in divided doses.

The above composition may be administered to various mammals including rats, livestock, humans, etc., via various routes. All possible administration routes may be considered, e.g., oral administration, rectal or intravenous administration, intramuscular administration, subcutaneous administration, endometrial administration or intracerebroventricular injections, etc.

Disclosed is a method for treating STAT3-mediated diseases including administering the above composition to a subject suspected of having the same.

Specifically, the method of treatment includes administering a pharmaceutically effective amount of the pharmaceutical composition to a subject suspected of having STAT3-mediated disease(s). The subject may refer to mammals including dogs, cows, horses, rabbits, mice, rats, chickens, or humans, but are not limited thereto. The pharmaceutical composition may be administered by parenteral, subcutaneous, intraperitoneal, intrapulmonary, and intranasal routes, and if necessary for local immunosuppressive treatment, it may be administered via an appropriate method including administration into the lesions. Parenteral administration may include intramuscular, intravenous, intra-arterial, intra-abdominal, or subcutaneous administrations. Preferable administration may include intravenous injection, subcutaneous injection, intradermal injection, muscular injection, and intravenous infusion. The preferable amount of the pharmaceutical composition to be administered may vary depending on the physical state and body weight of a subject, severity of illness, drug type, routes and duration of administration, but may be appropriately determined by a skilled person in the art. A subject suspected of having STAT3-mediated diseases can be treated by administering a pharmaceutical composition containing a *Salvia plebeia* R. Br. extract or fraction thereof as an active ingredient thereby preventing occurrence or progress of STAT3-mediated diseases. STAT3-mediated diseases are the same as explained above.

In another aspect of the present invention, there is provided a food composition for use in the prevention or improvement of STAT3-mediated diseases containing a *Salvia plebeia* R. Br. extract or fraction thereof as an active ingredient, wherein the STAT3-mediated disease is osteoporosis, rheumatoid arthritis, atopic dermatitis or Crohn's disease.

The *Salvia plebeia* R. Br. extract or fraction thereof are the same as explained above. More specifically, the *Salvia plebeia* R. Br. extract or fraction thereof for use according to the present invention may be added to a food composition for use in the prevention or improvement of STAT3-mediated diseases, wherein the STAT3-mediated disease is osteoporosis, rheumatoid arthritis, atopic dermatitis or Crohn's disease.

When the *Salvia plebeia* R. Br. extract or fraction thereof for use according to the present invention is used as a food additive the *Salvia plebeia* R. Br. extract or fraction thereof may be added as it is or in combination with other food(s) or food component(s), and may be appropriately used according to a conventional method. The mixed amount of the active ingredients may be appropriately determined according to the intended purposes of use.

The types of foods of the present invention are not particularly limited. Examples of the foods to which the *Salvia plebeia* R. Br. extract or fraction thereof may be added may include meats, sausages, bread, chocolates, candies, snacks, cookies, pizzas, ramen, noodles, gum, dairy products including ice creams, various kinds of soup, beverages, teas, drinks, alcoholic beverages, vitamin complexes, etc., all kinds of foods in a conventional sense, and foods used for animal feeds.

Additionally, the food composition for use according to the present invention may also contain various nutrients, vitamins, electrolytes, flavoring agents, colorring agents, pectic acid and its salts, alginic acid and its salts, organic acids, protective colloidal thickeners, pH adjusters, stabilizers, preservatives, glycerine, alcohols, carbonators used for carbonated beverages, etc. Additionally, the food composition for use according to the present invention may contain fruit flesh for the preparation of fruit juices, fruit juice beverages, and vegetable juices. Additionally, the food may be prepared in the form of tablets, granules, capsules, liquid solutions, pills, etc., according to a known manufacturing method. There is no other particular limitation regarding components of the food composition except containing the *Salvia plebeia* R. Br. extract or fraction thereof for use according to the present invention, and various conventional flavoring agents or natural carbohydrate, etc., may be further contained therein.

Additionally, the food composition for use according to the present invention may also contain various nutrients, vitamins, electrolytes, flavoring agents, coloring agents, pectic acid and its salts, alginic acid and its salts, organic acids, protective colloidal thickeners, pH adjusters, stabilizers, preservatives, glycerine, alcohols, carbonators used for carbonated beverages, etc. Additionally, the food composition for use according to the present invention may contain fruit flesh for the preparation of fruit juices, fruit juice beverages, and vegetable juices. The components may be used independently or in combination.

In another aspect of the present invention, there is provided a quasi-drug composition for use in the prevention or improvement of STAT3-mediated diseases containing a *Salvia plebeia* R. Br. extract or fraction thereof as an active ingredient, wherein the STAT3-mediated disease is osteoporosis, rheumatoid arthritis, atopic dermatitis or Crohn's disease.

The *Salvia plebeia* R. Br. extract or fraction thereof are the same as explained above. More specifically, the composition for use according to the present invention may be added to the quasi-drug composition for the prevention or improvement of STAT3-mediated diseases, wherein the STAT3-mediated disease is osteoporosis, rheumatoid arthritis, atopic dermatitis or Crohn's disease.

As used herein, "quasi-drugs" refers to a product corresponding to any one selected from a textile product, a rubber product, or an analogue thereof used for the purpose of treatment, alleviation, handling, or prevention of human or animal diseases; a product which, not being a tool, a machine, or an analogue thereof, has minimal effects or does not have any effect on humans; and a preparation used for the purpose of disinfection, pest control, or a similar use thereof for the prevention of infectious diseases, which, among the products being used for the purpose of treatment, alleviation, handling, or prevention of human or animal diseases, excludes those which are not a tool, a machine, or an analogue thereof; and which, among the products being used for the purpose of rendering a pharmacological effect on the human or animal structures and functions, excludes those which are not a tool, a machine, or an analogue thereof. Additionally, the above quasi-drugs include external skin preparations and personal hygiene products.

When the *Salvia plebeia* R. Br. extract or fraction thereof for use according to the present invention is added as an additive for the quasi-drugs the extract or fraction thereof may be added as it is, or used in combination with other quasi-drug(s) or component(s) therein, and can be appropriately used according to a conventional method. The mixed amount of the active ingredients may be appropriately determined according to the intended purposes of use.

The above external skin preparations, although not limited thereto, may be preferably formulated into ointments, lotions, sprays, patches, creams, powders, suspensions, gel preparations, or in the form of a gel. The individual hygiene products, although not limited thereto, may be preferably soaps, cosmetics, wet tissues, toilet paper, shampoos, skin creams, facial creams, toothpastes, lipsticks, fragrance, make-up, foundations, blushers, mascaras, eyeshadow, sunscreen lotions, hair treatment products, air-freshener gels, or cleansing gels. Additionally, examples of the quasi-drug composition for use according to the present invention may further include disinfecting cleaners, shower foams, gargles, wet tissues, hand washes, humidifier fillers, masks, ointments, or filter fillers.

### Detailed Description of the Invention

Hereinafter, the present invention will be described in more detail with reference to the following examples and experimental examples. However, the following examples and experimental examples are provided for illustrative purposes only, and the scope of the present invention should not be limited thereto in any manner.

### Example 1: Preparation of Salvia plebeia R Br. extract

### Example 1-1: Preparation of Salvia plebeia R. Br.

*Salvia plebeia* R. Br was thoroughly cleaned with water, dried in the shade, and powdered by a waring blender. Eight kg of the powdered *Salvia plebeia* R. Br was added with 50 L of ethanol, subjected to cold extraction at room temperature for 7 days, and filtered under reduced pressure via filter paper (Whatmann PLC, USA). The resulting filtered extract was subjected to a rotary vacuum evaporator at room temperature to remove ethanol therefrom, and 1.1 kg of *Salvia plebeia* R. Br crude extract was obtained as the extracted residue.

### Example 1-2: Preparation of fractions

In order to separate an active fraction from the above crude extract, the *Salvia plebeia* R. Br crude extract was suspended in 1 L of water, added with an equal volume of ethyl acetate and mixed for fractionation. The entire process was repeated 3 times to obtain 1 L of water-soluble fraction and 3 L of ethyl acetate-soluble fraction, and the ethyl acetate-soluble fraction was concentrated under reduced pressure to obtain 145 g of ethyl acetate-soluble extract. The remaining water-soluble fraction was concentrated under reduced pressure to obtain 35 g, and it was used as a water fraction.

### Example 2: Analysis of the inhibitory effect of Salvia plebeia R. Br. extract and fraction thereof on transcription activity of STAT3 induced by IL-6

In order to examine the inhibitory effect of *Salvia plebeia* R. Br. extract and fraction thereof against the transcription activity of STAT3 induced by IL-6, an experiment was performed as follows:

### Example 2-1: Preparation of a transformant introduced with luciferase

Hep3B cells (ATCC HB-8064) were transfected with *pStat3-Luc* containing a STAT3 reporter gene and *pcDNA3.1(*+*)* (Clontech laboratories, Palo Alto, CA, USA) using Lipofectamine PLUS^{®} (Invitrogen, Carlsbad, CA, USA). Two days after the transfection, the transfected cells were treated with hygromycin at a concentration of 100 µg/mL and obtained clones which stably expressed luciferase. The stable expression of luciferase on the clones was confirmed via luciferase assay.

### Example 2-2: Examination on IL-6 reactive STAT3 luciferase

The transfected cells were cultured in DMEM (GIBCO 119950965) medium under serum starvation, and the transfected cells was treated for 1 hour as shown below, added with 10 ng/mL IL-6 (R&D system, USA) and cultured for 12 hours.
1: Negative Control (Untreated Group);
2: Positive Control (IL-6, 10 ng/mL);
3: Extract and Fraction (1,3,6, and 10 µg/mL); and
4: Genestein-Treated Group (100 µM)

The above reacted cells were washed with PBS, added with 50µL of a buffer solution for dissolution (luciferase assay system, Promega, USA), stirred for 20 minutes, added with 30 - 100 µL of luciferase substrate (luciferase assay system, Promega, USA), and the level of color development was measured within 5 minutes using a luminometer (EG&G BERTHOLD, USA). The result revealed that the ethanol extract of *Salvia plebeia* R. Br. and an ethyl acetate fraction thereof inhibited the luciferase activity of STAT3 in a concentration-dependent manner. Specifically, the ethanol extract of *Salvia plebeia* R. Br. inhibited the luciferase activity at concentrations of 10, 6, 3, and 1 µg/mL by 88%, 73%, 48%, and 23%, respectively, and the ethyl acetate fraction inhibited the luciferase activity at concentrations of 6, 3, and 1 µg/mL by 100%, 98%, and 72%, respectively (FIG. 1).

The above results confirm that the *Salvia plebeia* R. Br. extract or fraction thereof can effectively inhibit the activation of STAT3 induced by IL-6.

STAT3 has been known to increase the expression of hepcidin, which is activated in inflammatory anemia and inhibits the iron release from macrophages (Wrighting DM et al., Blood. 2006 Nov 1; 108 (9): 3204-9), and is activated in inflammatory diseases such as human nephritis *(*Arakawa T et al., Nephrol Dial Transplant. 2008 Nov; 23 (11): 3418-26), skeletal muscle of cachexia patients, Crohn's disease *(*Lovato P et al., JBiol Chem. 2003 May 9; 278 (19): 16777-81), and chronic pancreatitis *(*Fukuda A et al., Cancer Cell. 2011 Apr 12; 19 (4): 441-55). Accordingly, the composition of the present invention containing *Salvia plebeia* R. Br. extract or fraction thereof can be useful for the prevention or treatment of the above inflammatory diseases.

Additionally, STAT3 has been considered a new target for bone resorption in rheumatoid arthritis. In the case of asthma, STAT3 present on the epithelium of airway has been known to induce allergic inflammatory reactions *(*Simeone-Penney MC et al., J Immunol. 2007 May 15; 178 (10): 6191-9), and STAT3 has been known to be activated by phosphorylation in occurrence of graft-versus-host disease (GVHD) (Ma HH et al., Cell Immunol. 2011; 268 (1): 37-46), multiple sclerosis patients and ApopE-/- mice with arteriosclerosis.

Accordingly, the above results confirm that the composition of the present invention containing *Salvia plebeia* R. Br. extract or fraction thereof can be useful for the prevention or treatment of autoimmune diseases and metabolic diseases.

### Example 2-3: Confirmation of inhibitory effect of Salvia plebeia R. Br. extract or fraction thereof on phosphorylation of STAT3, JAK2 and ERK induced by IL-6

Hep3B and U266 cells were aliquoted into a 6-well plate at a concentration of 5 x 10⁵ cells/well, cultured until they fill the culture plate at a density of 80%. Then, the medium was exchanged with serum starvation medium, cultured for additional 12 hours, and the transfected cells were treated for 60 minutes as shown below.

1: Negative Control (untreated group);
2: Positive Control (IL-16 10 ng/mL);
3: Extract and Fraction (10, 30, and 60 µg/mL); and
4: Genestein-Treated Group (100 µM)

Then, the resultant was treated with IL-6 at a concentration of 20 ng/mL and reacted for 20 minutes, lysed with 40 (µL of a buffer solution for dissolution [pH 8, 20 mM Tris-HCl, 137 mM NaCl, 10% glycerol, 1% Triton X-100, 1 mM Na₃VO₄, 2 mM EDTA, 1 mM PMSF, 20mM leupeptin, 20 mg/mL of aprotonin (Sigma, USA)], centrifuged (13000 g, 15 min), and the supernatant containing dissolved proteins was recovered. The concentration of the proteins was quantitated using a DC protein assay kit (Bio-Rad, USA), and the protein was loaded into a 4 - 12% SDS-polyacrylamide gel (SDS-PAGE), and an electrophoresis was performed at 175 mA for 2 hours. Upon completion of electrophoresis, the proteins in the gel were transferred onto a PVDF membrane (Westran S, pore size 0.2; Whatman, USA) at 35 V for 90 minutes. The transferred membrane was blocked at room temperature for 1 hour using Tris-buffer solution (T-TBS; 50 mM Tris-HCl, pH 7.6, 150 mM NaCl, 0.2% Tween-20, 5% skim milk; Sigma, USA), and washed 5 times with T-TBS. The above membrane was treated with multi-clone antibodies of phospho-Stat3, phospho-JAK2 and phospho-ERK (1:1000 dilution) as primary antibodies for 4 -12 hours. The resultant was washed 5 times with T-TBS, and allowed to react with HRP-coupled anti-mouse antibodies (1:5000 dilution) and anti-rabbit antibodies as secondary antibodies for 1 hour. The resultant was washed with T-TBS, and the film was developed in a dark room using ECL kit (Amersham, USA). As a result, it was confirmed that the ethanol extract of *Salvia plebeia* R. Br and an ethyl acetate fraction thereof inhibited the phosphorylation of STAT3, JAK2 and ERK induced by IL-6 (FIGS. 2-4).

The above results indicate that the *Salvia plebeia* R. Br extract or fraction thereof inhibits the phosphorylation of JAK2 in downstream of IL-6 thus resulting in its inactivation, and also reduces the phosphorylation of STAT3, being phosphorylated and activated by JAK2, that is, the *Salvia plebeia* R. Br extract or fraction thereof inhibits the signal transduction system mediated by IL-6. Furthermore, the above results support that the composition of the present invention containing the *Salvia plebeia* R. Br extract or fraction thereof as an active ingredient can be used for the prevention or treatment of diseases mediated by STAT3, which is activated by IL-6.

### Example 2-4: Confirmation of inhibitory effect of Salvia plebeia R. Br. extract or fraction thereof on MrNA expression of SOCS-3, MCP-1 and ICAM-1 induced by STAT3

Hep3B cells were aliquoted into a 6-well plate at a concentration of 5 x 10⁵ cells/well, cultured until they fill the culture plate at a density of 80%. Then, the medium was exchanged with serum starvation medium, cultured for an additional 12 hours, and the transfected cells were treated for 60 minutes as shown below.

1: Negative Control (untreated group);
2: Positive Control (IL-16,10 ng/mL); and
3: Extract and Fraction (10, 30, and 60 µg/mL);

Then, the cells were treated with IL-6 at a concentration of 20 ng/mL, reacted for 6 hours, and the resulting cells were collected into a tube and centrifuged to remove the medium, washed once with PBS, and RNA was extracted therefrom using RNeasy Mini Elute Cleanup kit. The concentration and purity of the extracted RNA were measured via 2100 Bioanalyzer system (Agilent Technologies), and cDNA was synthesized using Maxime RT PreMix (Random primer, iNtRON Biotechnology, INC).

The expression level of SOCS-3 mRNA was measured via Real-time PCR using TaqMan PCR master mix kit (Applied Biosystem). The expression level of MCP-1 and ICAM-1 mRNAs was measured via Real-time PCR using SYBRGreen master mix kit (Applied Biosystem).

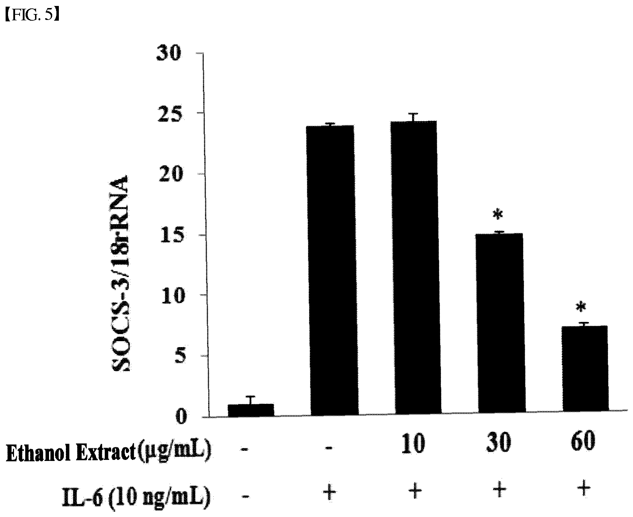

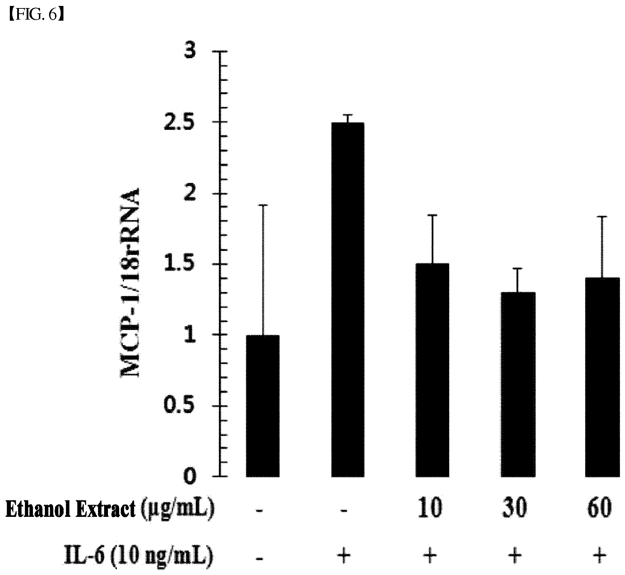

As a result, it was confirmed that the ethanol extract of *Salvia plebeia* R. Br inhibits the mRNA expression of SOCS-3 (a cytokine inhibitory factor), MCP-1 (cytokine), and ICAM-1 (intercellular adhesion molecule) (FIGS. 5 - 7). The results support that the *Salvia plebeia* R. Br extract or fraction thereof inhibits the signal transduction pathway of STAT3 activated by IL-6 based on the decrease of expression of target gene of STAT3.

### Example 3: Therapeutic effect of Salvia plebeia R. Br. extract on collagen-induced rheumatoid arthritis

In order to examine whether the *Salvia plebeia* R. Br extract has the therapeutic effect on rheumatoid arthritis by inhibiting rheumatoid arthritis induced by bovine type II collagen, an experiment was performed as follows:

### Example 3-1: Construction of a collagen-induced rheumatoid arthritis mouse model and confirmation of arthritis inhibitory effect

Rheumatoid arthritis, being a kind of autoimmune disease, is a chronic inflammatory disease characterized by the destruction and deformation of joints due to destruction of cartilages and bones caused by chronic hypertrophy and inflammatory reactions on synovial sheath of joints. In the present invention, the therapeutic effect of *Salvia plebeia* R. Br. extract of the present invention on collagen-induced arthritis was confirmed using a collagen-induced arthritis (CIA) model, as a representative animal model for rheumatoid arthritis.

Specifically, in the present invention, the collagen-induced arthritis mouse model was constructed using 6 - 8 week old BALB/c mice. The type II collagen (bovine CII, Chondrex) was mixed with a complete Freund adjuvant (Chondrex), and 100 µg of CII was intradermally injected onto the mouse tail. 21 days after the immunization, CII and the complete Freund adjuvant were mixed and subjected to secondary immunization.

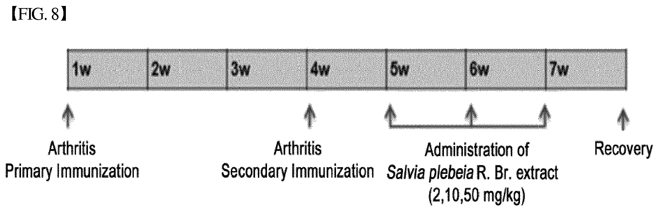

Then, in order to examine whether the *Salvia plebeia* R. Br. extract has a therapeutic effect on the arthritis-induced mice, the ethanol extract of *Salvia plebeia* R. Br. was dissolved in triple-distilled water at concentrations of 2 mg/kg, 10 mg/kg or 50 mg/kg, respectively, and orally administered daily starting from the 28^{th} day after the first immunization, and the control group was orally administered with 5 mg/kg of Ketoprofen (Sigma), the major component of Ketotop, which is the therapeutic agent for arthritis currently available in the market, after dissolving it in triple-distilled water. The above administration process is illustrated in FIG. 8, and `W' stands for 'week(s)' in FIG. 8. For clinical arthritis index, paw edema and erythema were evaluated as follows: 0 - 4 points (0: no edema, 1: slight edema in one joint, 2: moderate level of edema on at least 2 joints, 3: severe edema on most joints, and 4: overall severe edema). The experimental results are shown in FIGS. 9 -11.

As a result, as illustrated in FIGS. 9- 11, the oral administration of the ethanol extract of *Salvia plebeia* R. Br. was shown to effectively inhibit all of the paw thickness of type II collagen-induced rheumatoid arthritis mice, severity of arthritis, and incidence rate, and the effects were similar to those of Ketoprofen, a therapeutic agent for arthritis (FIGS. 9, 10, and 11).

### Example 3-2: Examination of blood immunoglobulins G1 and G2a

Upon completion of the experiment, mice were sacrificed, autopsied, and blood samples were collected from the abdominal region and centrifuged at 3,000 rpm for 15 minutes to separate blood sera. The concentrations of immunoglobulins G1 and G2a were measured using an ELISA kit (BD Biosciences) as follows:

Specifically, captured antibodies (purified rat anti-mouse IgG1 and anti-mouse IgG2a) were respectively diluted in a coating buffer (prepared by dissolving 8.40 g of NaHCO₃ and 3.56 g of Na₂CO₃ in 1 L of distilled water; pH 9.5) at a ratio of 1:250, aliquoted into each well (100 µL /well), reacted at 37°C for 3 hours, and washed 3 times with a wash buffer (0.05% Tween-20 PBS). Then, an assay diluent (10% FBS PBS) was aliquoted into each well (200 µL /well), reacted at 37°C for 1 hour, and washed 3 times with the wash buffer. The mouse blood serum was diluted in an assay diluents at a ratio of 1:300, and aliquoted into each well (100 µL /well), reacted at 37°C for 2 hours, and washed 5 times. Then, detection antibodies (biotin rat-anti mouse IgG1, bioninylated-anti-mouse IgG2a) and an enzyme reagent (SAv-HRP) were respectively diluted in the assay diluent at a ratio of 1:250, aliquoted into each well (100 (µL /well) as a working solution, reacted at room temperature for 1 hour, and respectively washed 7 times for 1 minute. Finally, a substrate solution was added to each well (100 µL/well), left therein for 30 minutes while being blocked from light, treated with 50 µL of stop solution. The resultant was measured of its absorbance at 450 nm via ELISA reader and the results are shown in FIGS. 12 and 13.

As a result, as illustrated in FIGS. 12 and 13, the *Salvia plebeia* R. Br. extract of the present invention was shown to effectively decrease the concentrations of IgG1 and IgG2a in blood (FIGS. 12 and 13).

### Example 4: Inhibitory effect of Salvia plebeia R. Br. extract on atopic dermatitis

In order to examine the inhibitory effect of *Salvia plebeia* R. Br. extract on atopic dermatitis an experiment was performed as follows:

### Example 4-1: Establishment of atopic dermatitis mouse model and confirmation of the effect of Salvia plebeia R. Br. extract on decrease of ear thickness

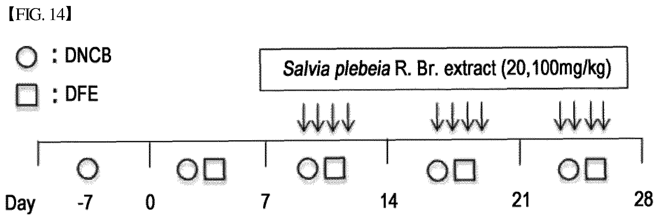

A house dust mite extract *(Dermatophagoides farinae* extract, DFE) and 2,4-dinitrochlorobenzene were used to induce atopic dermatitis in mice, and the process of inducing atopic dermatitis is illustrated in FIG. 14.

In order to confirm whether the *Salvia plebeia* R. Br. extract of the present invention can treat atopic dermatitis, a representative autoimmune disease, using the atopic dermatitis mouse model as described above, an experiment was performed as follows:

Specifically, an atopic dermatitis-induced mouse was orally administered with the *Salvia plebeia* R. Br. extract, and the effect of the *Salvia plebeia* R. Br. extract on the reduction of ear thickness of the atopic dermatitis-induced mouse was examined by an experiment performed as shown below.

1: Negative Control (Untreated Group);
2: Positive Control (DFE/DNCB 20 µg/mL);
3: Extract (20 mg/kg and 100 mg/kg)

Before applying a solution, the mouse ear was repeatedly striped 3 - 4 times with adhesive plaster under equal strength and rounds of turns, and the solution where DFE was dissolved at a concentration of 10 mg/mL was applied in the amount of 20 µg, respectively, every Thursday on the front/rear of the mouse ear. Additionally, DNCB (1%) dissolved in AOO (acetone/olive oil, 1:3) was applied on the front/rear of the mouse ear every Monday, and the ear thickness was measured on every Tuesday and Friday. As such, the application of DFE or DNCB on mouse skin increases its ear thickness, and the inhibitory effect of the *Salvia plebeia* R. Br. extract on the increase of ear thickness was examined.

As a result, as illustrated in FIG. 15, the oral administration of the ethanol extract of *Salvia plebeia* R. Br. was shown to decrease the ear thickness increase due to atopic dermatitis in a concentration-dependent manner (FIG. 15).

### Example 4-2: Confirmation of inhibitory effect of Salvia plebeia R. Br. extract on the amount of blood immunoglobulins E and G2a due to atopic dermatitis

Upon completion of the four-week experiment, the mice were sacrificed, and blood samples were collected therefrom, and centrifuged at 3,000 rpm for 15 minutes to separate blood sera of the mice. The concentrations of immunoglobulins E and G2a were measured using an ELISA kit (BD Biosciences) as follows:
Captured antibodies (purified rat anti-mouse IgG1 and anti-mouse IgG2a) were respectively diluted in a coating buffer (prepared by dissolving 8.40 g of NaHCO₃ and 3.56 g of Na₂CO₃ in 1L of distilled water; pH 9.5) at a ratio of 1:250, aliquoted into each well (100 µL, /well), reacted at 37°C for 3 hours, and washed 3 times with a wash buffer (0.05% Tween-20 PBS). Then, an assay diluent (10% FBS PBS) was aliquoted into each well (200 µL, /well), reacted at 37°C for 1 hour, and washed 3 times with the wash buffer. The mouse blood serum was diluted in an assay diluents at a ratio of 1:300, and aliquoted into each well (100 µL, /well), reacted at 37°C for 2 hours, and washed 5 times. Then, detection antibodies (biotin rat-anti mouse IgG1, bioninylated-anti-mouse IgG2a) and an enzyme reagent (SAv-HRP) were respectively diluted in the assay diluent at a ratio of 1:250, aliquoted into each well (100 µL, /well) as a working solution, reacted at room temperature for 1 hour, and respectively washed 7 times for 1 minute. Finally, a substrate solution was added to each well (100 µL/well), left therein for 30 minutes while being blocked from light, treated with 50 µL, of stop solution. The resultant was measured for its absorbance at 450 nm via ELISA reader and the results are shown in FIGS. 16 and 17.

As a result, as illustrated in FIGS. 16 and 17, the ethanol extract of *Salvia plebeia* R. Br. was shown to inhibit the amount of serum immunoglobulins E and G2a in a concentration-dependent manner (FIG. 15).

### Example 4-3: Measurement of inhibitory effect of Salvia plebeia R. Br. extract onhistamine release due to atopic dermatitis

blood sera of the mice were diluted in PBS to 1/100 in an Eppendorf tube, further added with 450 µL of 0.1 M HCl and 50 µL of 60% perchloric acid, and the mixture was centrifuged at 400 g for 20 minutes. Then, 800 µL of the resulting supernatant was recovered, added into a test tube containing 500 µL of 5M NaOH, 3 mL of distilled water, 10 mL of *n*-butanol, and 1.2 g of NaCl, stirred therein, and centrifuged at 500 g for 10 minutes. From the above test tube, 8 mL of butanol layer was collected, added with 3 mL of 0.1 M HCl and 10 mL of *n*-heptane and stirred, and centrifuged at 500 g for 10 minutes. 2 mL of the resulting aqueous layer was added with 400 µL of 1 M NaOH and 100 µL of 1% o-phthaldialdehyde solution (Sigma), left therein for 2 minutes, and its intensity of fluorescence at emission wavelength at 438nm, and excitation wavelength at 353 nm were measured via fluorescence spectrometer (RF-5301 PC, Shimadzu).

As a result, as illustrated in FIG. 18, the ethanol extract of *Salvia plebeia* R. Br. was shown to inhibit the histamine release in blood serum in a concentration-dependent manner, thus implying that the ethanol extract of *Salvia plebeia* R. Br. may have a therapeutic effect on atopic dermatitis, a representative autoimmune disease.

### Example 4-4: Confirmation of inhibitory effect of Salvia plebeia R. Br. extract on the secretion of inflammation-inducing cytokines due to atopic dermatitis

Upon completion of the experiment, mice were sacrificed and their ear tissues were cut out. In order to measure the expression of inflammation-inducing cytokines within the ear tissues, cDNA was synthesized using Maxime RT PreMix (Random primer, iNtRON Biotechnology). Each tube was added with 2 µL, of cDNA, 1 µL of sense and antisense primer solutions (0.4 µM), 12.5 (µL of SYBR Premix Ex Taq (Takarabio), and 9.5 µL, of dH₂O to a final volume of 25 µL. The expression level of TNF-α, IFN-γ, IL-4, IL-13, IL-31, and IL-17 mRNAs were measured via real-time PCR using TP850 software.

As a result, as illustrated in FIG. 19, the ethanol extract of *Salvia plebeia* R. Br. was shown to inhibit all the expressions of TNF-α, IFN-γ, IL-4, IL-13, IL-31, and IL-17, i.e., inflammation-inducing cytokines, in a concentration-dependent manner, thus being capable of exhibiting an inhibitory effect against their secretion.

Conclusively based on the above results, it was confirmed that the *Salvia plebeia* R. Br. extract has an excellent therapeutic effects for the prevention or treatment of both rheumatoid arthritis and atopic dermatitis, representative STAT3-mediated diseases, implying that the *Salvia plebeia* R. Br. extract can be used for the treatment of various STAT3-mediated diseases such as inflammatory diseases, autoimmune diseases, and metabolic diseases.

## Claims

1. A pharmaceutical composition for use in preventing or treating a STAT3-mediated disease comprising a *Salvia plebeia* R. Br. extract or fraction thereof as an active ingredient, wherein the STAT3-mediated disease is osteoporosis, rheumatoid arthritis, atopic dermatitis or Crohn's disease.

2. The pharmaceutical composition for use according to claim 1, wherein the extract is obtained by using the extraction solvent selected from the group consisting of water, C₁₋₆ alcohol, and a mixed solvent thereof.

3. The pharmaceutical composition for use according to claim 2, wherein the alcohol solvent is ethanol.

4. The pharmaceutical composition for use according to claim 1, wherein the fraction is an ethyl acetate fraction.

5. The pharmaceutical composition for use according to claim 1, wherein the composition is prepared into any one formulation selected from the group consisting of tablets, pills, powders, granules, capsules, suspensions, liquid medicine for internal use, emulsions, syrups, sterile aqueous solutions, non-aqueous solvents, lyophilized preparations, and suppositories.

6. A food composition for use in preventing or improving a STAT3-mediated disease comprising a *Salvia plebeia* R. Br. extract or fraction thereof as an active ingredient, wherein the STAT3-mediated disease is osteoporosis, rheumatoid arthritis, atopic dermatitis or Crohn's disease.

7. A quasi-drug composition for use in preventing or improving a STAT3-mediated disease comprising a *Salvia plebeia* R. Br. extract or fraction thereof as an active ingredient, wherein the STAT3-mediated disease is osteoporosis, rheumatoid arthritis, atopic dermatitis or Crohn's disease.

## Patentansprüche

1. Pharmazeutische Zusammensetzung zur Verwendung bei der Prävention oder Behandlung einer durch STAT3 vermittelten Erkrankung, wobei die Zusammensetzung einen *Salvia-plebeia-*R.-Br.-Extrakt oder eine Fraktion davon als Wirkstoff enthält, wobei die durch STAT3 vermittelte Erkrankung Osteoporose, rheumatoide Arthritis, atopische Dermatitis oder Morbus Crohn ist.

2. Pharmazeutische Zusammensetzung zur Verwendung nach Anspruch 1, worin der Extrakt unter Verwendung eines Extraktionslösungsmittels, ausgewählt aus der aus Wasser, C₁₋₆-Alkohol und einem Lösungsmittelgemisch davon bestehenden Gruppe erhalten wird.

3. Pharmazeutische Zusammensetzung zur Verwendung nach Anspruch 2, worin das Alkohollösungsmittel Ethanol ist.

4. Pharmazeutische Zusammensetzung zur Verwendung nach Anspruch 1, worin die Fraktion eine Ethylacetatfraktion ist.

5. Pharmazeutische Zusammensetzung zur Verwendung nach Anspruch 1, worin die Zusammensetzung in Form einer beliebigen Formulierung, ausgewählt aus der aus Tabletten, Pillen, Pulvern, Granulaten, Kapseln, Suspensionen, flüssiger Medizin zur inneren Anwendung, Emulsionen, Sirupen, sterilen wässrigen Lösungen, nicht wässrigen Lösungsmitteln, lyophilisierten Präparaten und Suppositorien bestehenden Gruppe hergestellt ist.

6. Nahrungsmittelzusammensetzung zur Verwendung zur Prävention oder Linderung einer durch STAT3 vermittelten Erkrankung, wobei die Zusammensetzung einen *Salvia-plebeia-*R.-Br.-Extrakt oder eine Fraktion davon als Wirkstoff enthält, wobei die durch STAT3 vermittelte Erkrankung Osteoporose, rheumatoide Arthritis, atopische Dermatitis oder Morbus Crohn ist.

7. Quasi-Arzneimittel zur Verwendung zur Prävention oder Linderung einer durch STAT3 vermittelten Erkrankung, wobei die Zusammensetzung einen *Salvia-plebeia-*R.-Br.-Extrakt oder eine Fraktion davon als Wirkstoff enthält, wobei die durch STAT3 vermittelte Erkrankung Osteoporose, rheumatoide Arthritis, atopische Dermatitis oder Morbus Crohn ist.

## Revendications

1. Composition pharmaceutique à utiliser pour la prévention ou le traitement d'une maladie à médiation par STAT3 comprenant un extrait de *Salvia plebeia* R. Br. ou une fraction de celui-ci comme ingrédient actif, dans laquelle la maladie à médiation par STAT3 est l'ostéoporose, l'arthrite rhumatoïde, la dermatite atopique ou la maladie de Crohn.

2. Composition pharmaceutique à utiliser selon la revendication 1, dans laquelle l'extrait est obtenu en utilisant le solvant d'extraction choisi dans le groupe comprenant l'eau, un alcool en C₁-₆ et un solvant mixte de ceux-ci.

3. Composition pharmaceutique à utiliser selon la revendication 2, dans laquelle le solvant alcoolique est l'éthanol.

4. Composition pharmaceutique à utiliser selon la revendication 1, dans laquelle la fraction est une fraction d'acétate d'éthyle.

5. Composition pharmaceutique à utiliser selon la revendication 1, dans laquelle la composition est préparée dans une quelconque formulation choisie dans le groupe comprenant comprimés, pilules, poudres, granules, capsules, suspensions, médicament liquide à usage interne, émulsions, sirops, solutions aqueuses stériles, solvants non aqueux, préparations lyophilisées, et suppositoires.

6. Composition alimentaire à utiliser pour la prévention ou l'amélioration d'une maladie à médiation par STAT3 comprenant un extrait de *Salvia plebeia* R. Br. ou une fraction de celui-ci comme ingrédient actif, dans laquelle la maladie à médiation par STAT3 est l'ostéoporose, l'arthrite rhumatoïde, la dermatite atopique ou la maladie de Crohn.

7. Composition quasi-médicamenteuse à utiliser pour la prévention ou l'amélioration d'une maladie à médiation par STAT3 comprenant un extrait de *Salvia plebeia* R. Br. ou une fraction de celui-ci comme ingrédient actif, dans laquelle la maladie à médiation par STAT3 est l'ostéoporose, l'arthrite rhumatoïde, la dermatite atopique ou la maladie de Crohn.
